# EUROPÄISCHE PATENTSCHRIFT

(11) **EP 1 177 011 B1**
(45) Veröffentlichungstag und Bekanntmachung des Hinweises auf die Patenterteilung: **17.11.2010**
(21) Anmeldenummer: 00931105.1
(22) Anmeldetag: 03.05.2000
(51) Int. Cl.: A61N 1/36

(54) **RETINA-IMPLANTAT**
RETINA IMPLANT
IMPLANT RETINIEN

(30) Priorität: 07.05.1999 DE 19921399
(43) Veröffentlichungstag der Anmeldung: 06.02.2002
(73) Patentinhaber: Retina Implant AG, 72770 Reutlingen (DE)
(72) Erfinder: NISCH, Wilfried, D-72072 Tübingen (DE); STETT, Alfred, D-72770 Reutlingen (DE); SCHUBERT, Markus, D-72074 Tübingen (DE); GRAF, Michael, D-71229 Leonberg (DE); GRAF, Heinz, Gerhard, D-71106 Magstadt (DE); HÄMMERLE, Hugo, D-72072 Tübingen (DE); ZRENNER, Eberhart, D-72076 Tübingen (DE); STELZLE, Martin, D-72766 Reutlingen (DE)
(74) Vertreter: Witte, Weller & Partner
(86) Internationale Anmeldenummer: PCT/EP2000/003957
(87) Internationale Veröffentlichungsnummer: WO 2000/067838

(56) Entgegenhaltungen:
- EP-A- 0 460 320
- WO-A-96/39221
- DE-A- 19 705 987
- US-A- 4 628 933
- US-A- 5 109 844

## Beschreibung

Die Erfindung betrifft ein Retina-Implantat mit einer Oberfläche, an der eine Mehrzahl von Pixelelementen zur Aufnahme und Umwandlung von einfallender Lichtenergie in elektrische Energie vorgesehen ist, mit mindestens einem Verstärker und mit einer Mehrzahl von Stimulationselektroden, die über den mindestens einen Verstärker in Abhängigkeit von den von den Pixelelementen aufgenommenen Signalen angesteuert werden.

Ein derartiges Retina-Implantat ist aus der US-A-4 628 933 bekannt.

Das bekannte Retina-Implantat ist ein sogenanntes epiretinales Implantat, das auf die Oberfläche der Netzhaut aufgesetzt wird. Es besteht im wesentlichen aus einem Chip, auf dem eine Vielzahl von lichtempfindlichen Elementen an einer der Linse des Auges zugewandten Oberfläche vorgesehen ist, durch die ein durch die Linse projiziertes Bild in entsprechende elektrische Signale umgewandelt wird, die die an der Retina befindlichen Photorezeptoren stimulieren sollen. Hierzu können die einzelnen von den lichtempfindlichen Elementen aufgenommenen elektrischen Signale verstärkt werden, um zugeordnete Stimulationselektroden an der Rückseite des Chips anzusteuern, die zur Stimulation der Zellen an der Retina dienen.

In neuerer Zeit geht die Entwicklung wegen systembedingter Nachteile weg von den epiretinalen Implantaten hin zu subretinalen Implantaten, die demzufolge in untere Schichten der Retina implantiert werden sollen. Ein derartiges subretinales Implantat ist beispielsweise aus der WO 98/17343 bekannt.

Das bekannte subretinale Retina-Implantat besitzt an seiner der Linse zugewandten Oberfläche eine Vielzahl von lichtempfindlichen Pixelelementen, die das über die Linse einfallende Bild in entsprechende elektrische Signale umwandeln und mit Unterstützung von zusätzlich durch das Auge eingestrahlter Infrarotstrahlung, die zur Energieversorgung über eine hinter den Pixelelementen liegende photovoltaische Schicht dient, in elektrische Stimulussignale umsetzt. Diese werden über Stimulationselektroden, die sich gleichfalls an der der Linse zugewandten Oberfläche des Implantats befinden, an die anliegenden Zellen der Retina abgegeben.

Sowohl bei den epiretinalen als auch bei den subretinalen Implantaten hat es sich gezeigt, daß die Einkopplung von externer Energie in die Implantate notwendig ist, um die einfallenden Lichtsignale in entsprechende elektrische Stimuli für eine ausreichende Stimulation der am Implantat anliegenden Zellen umzusetzen. Neben der Einkopplung von nicht sichtbarer Infrarotstrahlung sind verschiedene weitere Möglichkeiten der Energieeinkopplung bekannt, wozu etwa die Möglichkeit der Einkopplung von HF-Energie über eine Spule oder die Verwendung einer externen oder einer implantierten Batterie gehören.

Als ein Problem hat sich bei den bekannten Retina-Implantaten gezeigt, daß es schwierig ist, die einfallende Lichtenergie weitgehend linear über einen großen Intensitätsbereich, der bei natürlichen Lichtschwankungen mehrere Zehnerpotenzen umfaßt, in entsprechende elektrische Stimulussignale umzusetzen.

Die Aufgabe der Erfindung besteht demnach darin, ein verbessertes Retina-Implantat anzugeben, das eine Umsetzung der einfallenden Lichtenergie über einen möglichst großen Intensitätsbereich in geeignete elektrische Stimulussignale ermöglicht.

Diese Aufgabe wird bei einem Retina-Implantat gemäß der eingangs genannten Art erfindungsgemäß dadurch gelöst, daß mindestens ein lichtempfindliches Referenzelement, das eine größere Oberfläche aufweist als ein Pixelelement, oder dass eine Mehrzahl von Referenzelementen vorgesehen sind, wobei das Referenzelement oder die Mehrzahl von Referenzelementen mit dem zumindestens einen Verstärker gekoppelt ist, um dessen Verstärkung in Abhängigkeit von der auf das mindestens eine Referenzelement einfallenden Lichtenergie zu steuern.

Auf diese Weise können die abgegebenen elektrischen Stimulussignale an eine durchschnittliche Lichtintensität angepaßt werden, wie dies auch bei der natürlichen Adaption des Auges an veränderte Lichtverhältnisse erfolgt. Es wird somit einerseits vermieden, daß bei relativ hellem Umgebungslicht über die Stimulationselektroden zu starke elektrische Signale an die anliegenden Zellen der Retina übertragen werden, die zu einer Überreizung oder gar zu Schäden an diesen Zellen führen könnten, andererseits wird es auch bei sehr schwachen Lichtbedingungen ermöglicht, Stimulussignale ausreichender Stärke an die anliegenden Zellen der Retina zu übertragen, so daß einem Patienten eine deutlich verbesserte Sehhilfe auch bei veränderlichen Umgebungsbedingungen geboten werden kann.

Das mindestens eine Referenzelement sollte hierbei naturgemäß entweder zumindest eine Oberfläche aufweisen, die größer ist als die Oberfläche eines einzelnen Pixelelementes, oder aber es sollte eine Mehrzahl von Referenzelementen vorgesehen sein, die eine Mittelwertbildung über einen ausgewählten Bereich der Oberfläche ermöglichen. Über das mindestens eine Referenzelement wird also der Arbeitsbereich der einzelnen Pixelelemente festgelegt bzw. die Verstärkungskennlinie in Abhängigkeit von der mittleren Beleuchtungsstärke verschoben.

Die Größe des mindestens einen Referenzelementes hängt davon ab, über welchen Bereich die mittlere Beleuchtungsstärke ermittelt werden soll. Die Größe kann deshalb zwischen wenigen µm bis zu einigen mm² reichen.

In vorteilhafter Weiterbildung der Erfindung ist ein gemeinsames, sich über einen ausgewählten Bereich der Oberfläche erstreckendes Referenzelement vorgesehen, auf dem Pixelelemente als isolierte Bereiche, vorzugsweise in einem Gittermuster, angeordnet sind.

Auf diese Weise ergibt sich ein relativ einfacher Aufbau und eine Anpassungsmöglichkeit an die gesamte durchschnittliche Beleuchtungsstärke.

Gemäß einer weiteren Ausgestaltung der Erfindung ist eine Mehrzahl von Referenzelementen über einen ausgewählten Bereich der Oberfläche verteilt in einem vorgegebenen Muster zwischen den Pixelelementen angeordnet.

Auf diese Weise läßt sich eine spezielle Charakteristik bei der Ermittlung der mittleren Beleuchtungsstärke erreichen, die an persönliche physiologische Bedingungen des Patienten angepaßt werden kann.

Gemäß einer Weiterbildung dieser Ausführung sind die Referenzelemente als Streifen und/oder als rechtwinkliges oder schiefwinkliges Gitternetz über einen ausgewählten Bereich der Oberfläche verteilt angeordnet.

Mit einem derartigen Aufbau lassen sich verschiedene Variationsmöglichkeiten bei der Ermittlung der mittleren Beleuchtungsstärke erreichen, die einerseits einen einfachen Aufbau und andererseits eine optimale Anpassung an vorgegebene Parameter ermöglichen.

Je nach Anzahl, Anordnung und Verschaltung der Referenzelemente kann jeweils ein Referenzelement einer Mehrzahl von Pixelelementen zugeordnet sein, jedem Pixelelement ein Referenzelement zugeordnet sein oder auch einem Pixelelement mehrere Referenzelemente zugeordnet sein.

Bei einer weiteren bevorzugten Ausführung der Erfindung ist jedem Pixelelement ein Verstärker und eine Stimulationselektrode zugeordnet.

Auf diese Weise könnend die von den einzelnen Pixelelementen aufgenommenen Lichtsignale mit möglichst hoher Auflösung zur Stimulation der anliegenden Zellen genutzt werden.

Gemäß einer Weiterbildung der Erfindung sind die Referenzelemente als Streifen über einen ausgewählten Bereich der Oberfläche verteilt angeordnet, wobei zwischen einzelnen Streifen und Pixelelementen weitere Referenzelemente angeordnet sind.

Auch hierdurch wird eine besonders gute Anpassungsmöglichkeit an vorgegebene externe oder physiologische Parameter des Patienten ermöglicht.

Gemäß einer weiteren Ausführung der Erfindung ist eine Mehrzahl von Referenzelementen schachbrettartig über einen ausgewählten Bereich der Oberfläche verteilt angeordnet, wobei jedes Pixelelement von mehreren Referenzelementen umgeben ist, die winklig zueinander angeordnet sind.

Hierbei können die Referenzelemente je nach gewählter geometrischer Anordnung eine größere Länge oder Breite als das zugeordnete Pixelelement aufweisen oder auch in einzelne Segmente unterteilt sein, die einem einzelnen Pixelelement zugeordnet sind.

Auf diese Weise ist es möglich, die ausgewählten Bereiche der Oberfläche, die zur Ermittlung des Referenzsignals für ein einzelnes Pixelelement benötigt werden, überlappen zu lassen. Hierbei können auch mehrere Referenzelemente bzw. mehrere Segmente eines Referenzelementes einem Pixelelement zugeordnet werden, um die mittlere Beleuchtung über einen größeren Bereich der Oberfläche zu ermitteln. Andererseits können über einen größeren ausgewählten Bereich der Oberfläche verteilte einzelne Referenzelemente auch mehreren Pixelelementen oder dem ganzen Retina-Implantat als Referenz dienen.

Auf diese Weise entstehen Chipsegmente, die die gesamte Oberfläche des Retina-Implantats zur Bestimmung der mittleren Beleuchtungsstärke nutzen, wobei bei einem Ausfall eines Referenzelementes nur ein Teil des Retina-Implantats seine Funktion verlieren würde.

Gemäß einer weiteren Ausgestaltung der Erfindung verstärkt der mindestens eine Verstärker die Differenz zwischen den Ausgangssignalen der Pixelelemente und der zugeordneten Referenzelemente und steuert die zugeordneten Stimulationselektroden in Abhängigkeit von den verstärkten Signalen.

Auf diese Weise ergibt sich ein besonders einfacher und zuverlässiger Aufbau der Verstärkerschaltung. Zusätzliche Maßnahmen zur Mittelwertbildung und dergleichen sind nicht erforderlich.

Gemäß einer weiteren Ausgestaltung der Erfindung weisen die Pixelelemente und das mindestens eine Referenzelement eine gleiche Wellenlängenempfindlichkeit auf.

Auf diese Weise kann das mindestens eine Referenzelement unmittelbar zur Festlegung des Arbeitsbereiches des jeweiligen Verstärkers verwendet werden.

Gemäß einer weiteren Ausgestaltung der Erfindung sind die Pixelelemente und das mindestens eine Referenzelement als Photodioden ausgebildet.

Hierzu sind geeignete Herstellungsverfahren bekannt, die eine geordnete Anordnung einer hohen Anzahl von Pixelelementen in einem Array mit hoher Packungsdichte ermöglichen.

Der oder die Verstärker können eine lineare oder logarithmische Kennlinie aufweisen.

Insbesondere die Verwendung von Verstärkern mit logarithmischer Kennlinie kann von Vorteil sein, da sich selbst bei einer Variation der Eingangssignale über einen großen Intensitätsbereich die Ausgangssignale nur in einem relativ geringen Umfang ändern.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Weitere Merkmale und Vorteile der Erfindung ergeben sich aus der nachfolgenden Beschreibung bevorzugter Ausführungsbeispiele unter Bezugnahme auf die Zeichnung. Es zeigen:
- Fig. 1: einen äußerst schematisierten Querschnitt durch ein Auge, dessen Retina mit einem erfindungsgemäßen Im- plantat versehen ist;
- Fig. 2: eine schematische Darstellung eines Ausschnitts einer möglichen Ausführung des erfindungsgemäßen Retina-Implantats subretinaler Anordnung in stark vergrößertem Maßstab in quergeschnittener Darstel- lung;
- Fig. 3: eine schematische Darstellung einer Aufsicht auf die Oberfläche des Retina-Implantats, aus der eine erste mögliche Anordnung von Pixelelementen, Verstärkern mit Stimulationselektroden und einer gemeinsamen Re- ferenzelement sichtbar ist;
- Fig. 3a: eine schematische Darstellung einer weiteren gegen- über der Ausführung gemäß Fig. 3 abgewandelten An- ordnung von Pixelelementen, Elektroden und Referenz- elementen;
- Fig. 3b: eine weitere Abwandlung der Ausführung gemäß Fig. 3a;
- Fig. 3c: eine weitere Abwandlung der Ausführung gemäß Fig. 3b; und
- Fig. 4: eine schematische Darstellung des Prinzips der Er- findung anhand einer Verschiebung der Verstärker- kennlinie in Abhängigkeit von der mittleren Beleuch- tungsstärke.

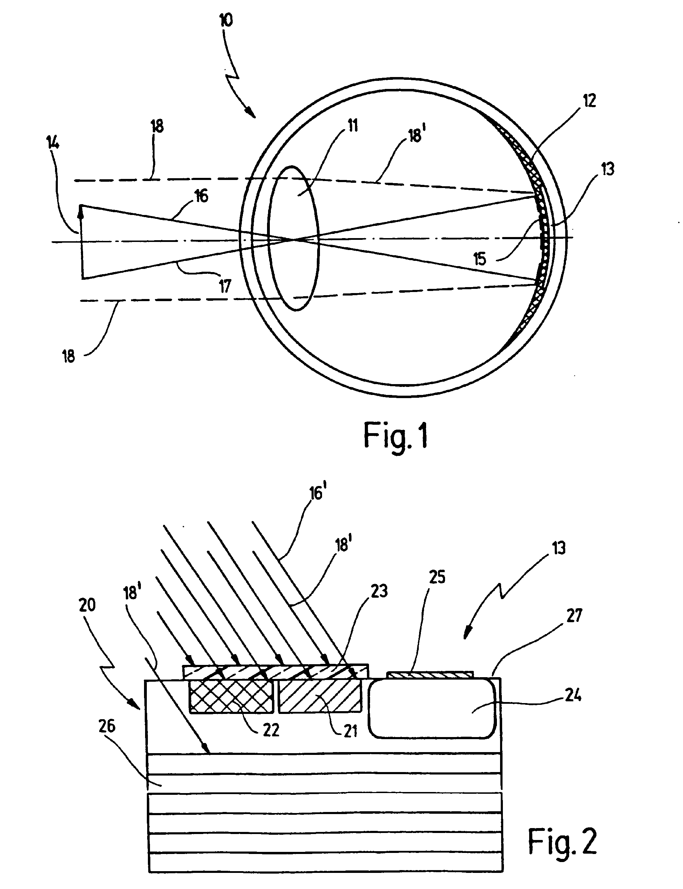

In Fig. 1 ist mit der Ziffer 10 insgesamt ein Auge, beispielsweise ein menschliches Auge, bezeichnet. Im Auge 10 sind schematisch eine Linse 11 und eine Retina 12 angedeutet.

Mit 13 ist ein subretinales Implantat zu erkennen, das sich in den unteren Schichten der Retina 12 befindet.

Ein Gegenstand 14 wird in der üblichen Weise durch die Linse 11 auf der Retina 12 abgebildet, wie mit Strahlen 16, 17 und einem auf dem Kopf stehenden Bild 15 angedeutet ist.

Mit 18 ist in Fig. 1 ein Bündel von Strahlen eines nicht sichtbaren Lichtes, z.B. eines Infrarotlichtes, angedeutet, wobei das Lichtbündel 18 so ausgelegt ist, daß es das Implantat 13 vollflächig trifft. Hierzu wird das Lichtbündel 18 durch die Linse 11 lediglich geringfügig verändert, wie mit 18' im Auge 10 angedeutet. Eine solche Möglichkeit zur externen Energieeinkopplung zur Stromversorgung des Retina-Implantats 13 ist grundsätzlich aus der WO 98/17343 bekannt.

Das erfindungsgemäße Implantat 13 zeichnet sich nun durch einen besonderen Aufbau aus, wie dieser beispielhaft anhand der nachfolgenden Figuren 2 bis 4 erläutert wird.

Es versteht sich, daß die Möglichkeit der hier beschriebenen Energieeinkopplung zur Stromversorgung durch Infrarotenergie nicht beschränkend zu verstehen ist, und daß sämtliche bekannten Möglichkeiten zur Energieeinkopplung verwendet werden können, also insbesondere auch die Möglichkeit zur induktiven Einkopplung über HF-Energie.

An der der Linse 11 zugewandten aktiven Oberfläche 27 des Implantats 13 befindet sich nun eine Vielzahl von lichtempfindlichen Pixelelementen 21, die einfallende Lichtenergie, die in Fig. 2 beispielhaft mit den Pfeilen 16' angedeutet ist, in entsprechende elektrische Signale umwandeln.

Zur Stromversorgung dient die einfallende Infrarotstrahlung, die beispielhaft mit 18' angedeutet ist und von einem darunterliegenden Infrarot-Photodiodenstack in elektrische Energie umgesetzt wird, die zur Versorgung einer Verstärkerschaltung 24 dient, über die in Abhängigkeit von der einfallenden Lichtenergie eine Stimulationselektrode 25 zur Stimulierung der anliegenden Retina-Zellen angesteuert wird.

Erfindungsgemäß ist zusätzlich zu dem lichtempfindlichen Pixelelement 21 mindestens ein lichtempfindliches Referenzelement 22 vorgesehen, das schematisch in Fig. 2 neben dem Pixelelement 21 dargestellt ist.

Während bei herkömmlichen Anordnungen gemäß der WO 98/17343 das von einem Pixelelement 21 aufgenommene elektrische Signal unmittelbar in ein entsprechendes Stimulussignal der Stimulationselektrode 25 umgesetzt wurde, wird nunmehr mit Hilfe des Referenzelementes 22 über einen vorgegebenen ausgewählten Bereich der Oberfläche 27 eine mittlere Beleuchtungsstärke ermittelt, die zur Anpassung der Kennlinie des Verstärkers dient, über den die Stimulationselektrode 25 angesteuert wird.

Das Referenzelement 22 und das Pixelelement 21 sind bei der Ausführung gemäß Fig. 2 ferner in an sich bekannter Weise durch ein Filter 23 abgedeckt, das die Infrarotstrahlung 18' abblockt.

Wiederum sei darauf hingewiesen, daß diese Ausführung lediglich beispielhafter Natur ist und daß statt dessen auch das Prinzip der selektiven Absorption (also ohne eine zusätzliche Filterschicht) verwendet werden kann, oder daß grundsätzlich andere Arten der Energieeinkopplung genutzt werden können.

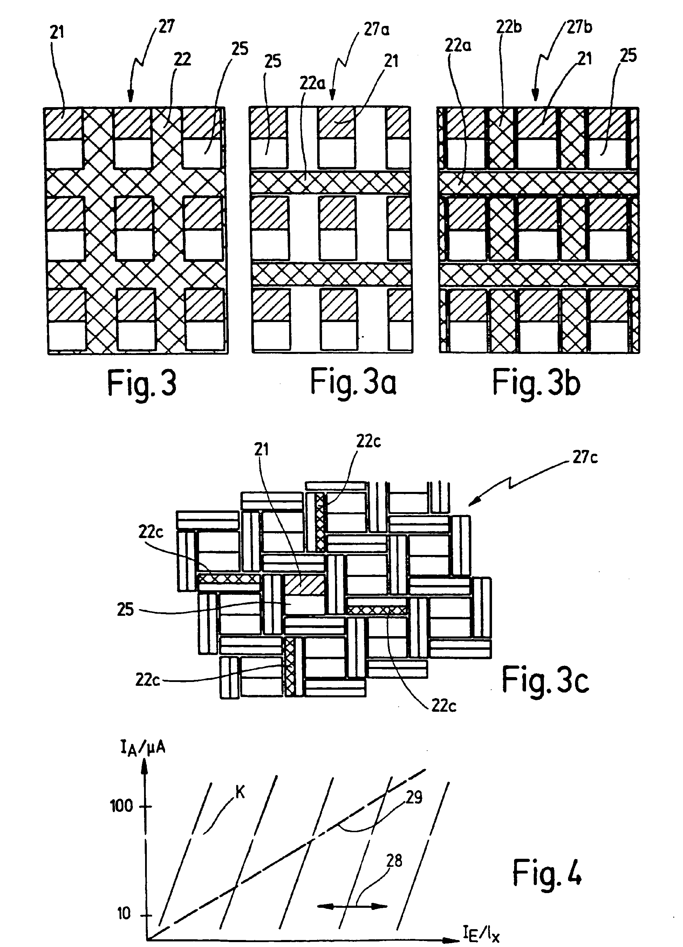

Das Grundprinzip der Erfindung ist unabhängig von dieser Anordnung und ist aus Fig. 4 näher zu ersehen.

In Fig. 4 ist die Ausgangsstromstärke I_{A} eines von einer Stimulationselektrode 25 abgegebenen Ausgangssignals in Abhängigkeit von der einfallenden Lichtintensität I_{E} in Lux angegeben.

Während eine herkömmliche Anordnung etwa gemäß der US-A-4 628 933 eine etwa lineare Übertragungskennlinie aufweisen kann, die in Fig. 4 beispielhaft durch die gestrichelte Linie 29 angedeutet ist, wird gemäß der Erfindung die Kennlinie K des Verstärkers 24 in Abhängigkeit von der mittleren Beleuchtungsstärke, die vom Referenzelement 22 registriert wird, an veränderte Lichtbedingungen angepaßt, d.h. verschoben, wie durch den Pfeil 28 angedeutet ist. Eine niedrige mittlere Lichtintensität führt zu einer Verschiebung der Kennlinie K nach links, während eine hohe mittlere Lichtintensität zu einer Verschiebung der Kennlinie K nach rechts führt.

Dies kann auf einfache Weise dadurch erreicht werden, daß der Verstärker 24 die Signaldifferenz zwischen dem Pixelelement 21 und dem Referenzelement 22 ermittelt und verstärkt. Das Referenzelement 22 definiert somit den Arbeitsbereich der Schaltung, so daß innerhalb eines Pixelelements das Ausgangssignal des Verstärkers 24 nur davon abhängt, wie stark die Beleuchtungsstärke des Pixelelements 21 von der des Referenzelements 22 abweicht. Es versteht sich, daß daher entweder das Referenzelement 22 eine größere Oberfläche als das Pixelelement 21 hat und/oder daß eine Mehrzahl von Referenzelementen 22 zur Ermittlung einer mittleren Beleuchtungsstärke verwendet wird.

Die Verstärkung muß sich hierbei natürlich nicht auf die Stromstärke oder Spannung beschränken, sondern kann auch auf aus diesen Größen abgeleitete Größen angewendet werden, wobei der Verstärkungsfaktor gering sein kann oder auch sehr hoch bis zu zehntausendfach oder mehr sein kann. Demgemäß kann der Verstärker 24 einstufig oder auch mehrstufig aufgebaut sein. Ferner kann die Übertragungskennlinie des Verstärkers 24 wie in Fig. 4 dargestellt linear oder auch andersgeartet sein, etwa logarithmisch von der Beleuchtungsstärke abhängen. Sie kann ferner auf einen kleinen Helligkeitsbereich beschränkt sein oder aber auch bis zu zehn Helligkeitsdekaden oder mehr übersteigen. Das Ausgangssignal des Verstärkers 24 kann sowohl zeitlich kontinuierlich sein, als auch durch eine zusätzliche Schaltung oder externe Einrichtungen getaktet werden. Das Ausgangssignal kann in Form von Pulsfolgen übermittelt werden oder auch als kontinuierliches Signal.

Grundsätzlich kann jedem Pixelelement 21 ein Verstärker 24 und auch eine Stimulationselektrode 25 zugeordnet sein, um eine möglichst gute Auflösung zu erreichen. Daneben ist es natürlich auch denkbar, mehrere Pixel an einen Verstärker 24 zu koppeln oder mehrere Pixelelemente 21 an eine Stimulationselektrode 25.

Bei dem beispielhaft anhand von Fig. 13 erläuterten Aufbau des erfindungsgemäßen Retina-Implantats 13 kann ein Chip 20, auf dem sich eine Vielzahl von Pixelelementen 21, Verstärkern 24, Stimulationselektroden 25 zusammen mit mindestens einer oder mehreren Referenzelementn 22 befindet, in an sich bekannter Weise in CMOS-Technik unter Verwendung von Lithographieverfahren hergestellt werden.

Da dies nicht Gegenstand der vorliegenden Erfindung ist, wird hierauf nicht näher eingegangen.

Anhand der Figuren 3 und 3a bis 3c werden nunmehr verschiedene mögliche Anordnungen von Pixelelementen, Referenzelementen und Stimulationselektroden erläutert, die lediglich beispielhaft zu verstehen sind.

In Fig. 3 ist in stark vergrößerter Darstellung ein Ausschnitt der aktiven Oberfläche 27, d.h. der Oberfläche, die mit wirksamen Schaltungselementen, d.h. mit Pixelelementen und Stimulationselektroden, versehen ist, in der Aufsicht schematisch dargestellt. Bei dieser ersten Ausführung ist ein einziges, durchgehendes Referenzelement 22 vorgesehen, auf dem jeweils Paare von Pixelelementen 21 mit angrenzender Stimulationselektrode 25, unterhalb derer jeweils ein zugeordneter Verstärker 24 vorgesehen ist, in regelmäßigen Abständen in einem Gittermuster nebeneinander angeordnet sind, wobei die jeweiligen Pixelelemente 21 und Stimulationselektroden 25 natürlich gegenüber dem Referenzelement 22 elektrisch isoliert sind, um Störungen der Funktion der Pixelelemente 21 und der Stimulationselektroden 25 zu vermeiden.

Bei der Ausführung gemäß Fig. 3a sind auf der aktiven Oberfläche 27a streifenförmige Referenzelemente 22a über einen ausgewählten Bereich der aktiven Oberfläche 27a angeordnet. Zwischen benachbarten streifenförmigen Referenzelementen 22a sind jeweils Paare von Pixelelementen 21 und Stimulationselektroden 25 mit zugehörigem Verstärker 24 unter Bildung von Abständen zwischen benachbarten Paaren angeordnet.

Hierbei ist jeweils ein Referenzelement 22a mit mehreren Pixelelementen 21 über den jeweiligen zugeordneten Verstärker 24 gekoppelt.

In Fig. 3b ist eine weitere Anordnung der Pixelelemente 21 und Referenzelemente 22a, 22b schematisch dargestellt. Hierbei sind auf der aktiven Oberfläche 27b wiederum streifenförmige Referenzelemente 22a in regelmäßigen Abständen voneinander über einen ausgewählten Teilbereich der aktiven Oberfläche 27 angeordnet und jeweils Paare von Pixelelementen 21 und Stimulationselektroden 25 zwischen diesen streifenförmigen Referenzelementen 22a unter Bildung von Abständen zueinander nebeneinander angeordnet. Dabei ist zwischen benachbarten Paaren von Pixelelementen 21 und Stimulationselektroden 25 jeweils ein weiteres rechteckförmiges Referenzelement 22b vorgesehen.

Dabei sind verschiedene Varianten der Verschaltung von Referenzelementen 22a, 22b und Verstärkern 24 möglich.

In Fig. 3c ist eine weitere beispielhafte Ausführung der Anordnung von Pixelelementen 21, Stimulationselektroden 25 mit zugehörigem Verstärker 24 und von Referenzelementen 22c dargestellt.

Über die aktive Oberfläche 27c verteilt sind eine Mehrzahl von Referenzelementen 22c schachbrettartig über einen ausgewählten Bereich der Oberfläche 27c verteilt angeordnet. Dabei ist jeweils ein Pixelelement 21 mit einer Stimulationselektrode 25 zu einem Paar verbunden, das eine quadratische Oberfläche aufweist und jeweils von einem Paar von Referenzelementen 22c umgeben ist, wobei die einzelnen Referenzelemente 22c schmaler sind, jedoch eine größere Länge aufweisen, so daß insgesamt vier Paare von Referenzelementen 22c mit einem seitlichen Überstand um ein aus Pixelelement 21 und zugehöriger Stimulationselektrode 25 gebildetes Quadrat herum derart angeordnet sind, so daß sich vom Umriß her wiederum ein durch die Paare von Referenzelementen 22c nach außen abgeschlossenes Quadrat ergibt. Dabei können die Außenseiten jeweils eines Paares von Referenzelementn 22c mit benachbarten Paaren von Referenzelementen 22 und Paaren von Pixelelementen 21 und Stimulationselektroden 25 sich wiederum zu Quadraten ergänzen, die versetzt gegenüber dem zuvor erwähnten Quadrat angeordnet sind.

Insgesamt entsteht auf diese Weise ein regelmäßiges Muster von Pixelelementen 21, Stimulationselektroden 25 und Referenzelementen 22c, bei denen jeweils ein aus Pixelelement 21 und Stimulationselektrode 25 gebildetes Quadrat von insgesamt vier mit seitlichem Überstand über das Quadrat hinaus angeordneten, sich wiederum zu einem größeren Quadrat ergänzenden Paaren von Referenzelementen 22c umgeben ist.

Die Zuordnung zwischen den jeweiligen Pixelelementen 21 und den Referenzelementen 22c kann nun auf verschiedene Arten variiert werden, um verschiedene Varianten bei der Ermittlung der mittleren Beleuchtungsstärke zu realisieren.

In Fig. 3c sind beispielhaft insgesamt vier Referenzelemente 22c mit einem Rastermuster gekennzeichnet, die zusammen mit einem in der Mitte liegenden Paar aus Pixelelement 21 und Stimulationselektrode 25 ein sternförmiges Muster mit Pixelelement 21 und Stimulationselektrode 25 im quadratischen Zentrum und vier hiervon nach außen weisenden, zueinander jeweils um 90° winkelmäßig versetzten dünnen rechteckförmigen Referenzelementen 22c bilden. Bei dieser Verschaltung können die vier Referenzelemente 22c dem Verstärker 24 eines Pixelelementes 21 zugeordnet sein. Auf diese Weise läßt sich das Ausgangssignal der Stimulationselektrode 25 über den zugeordneten Verstärker 24 in Abhängigkeit von einer mittleren Beleuchtungsstärke steuern, die über eine gewisse, örtlich begrenzte Umgebung in der Nähe des Pixelelementes 21 ermittelt wird. Des weiteren ist es denkbar, diese Referenzelemente 22c auch zur Steuerung von mehreren Stimulationselektroden 25 zu verwenden. Auch könnten diese Referenzelemente 22c oder eine andere Teilmenge der dargestellten Referenzelemente 22c zur Bestimmung der mittleren Beleuchtungsstärke für den gesamten Chip 20 oder für einen Teilbereich davon verwendet werden.

Auf diese Weise entstehen Chipsegmente, die die gesamte Fläche des Chips 20 zur Bestimmung der mittleren Beleuchtungsstärke nutzen, wobei bei einem Ausfall eines Referenzelementes nur ein Teil des Chips 20 seine Funktion verlieren würde.

In Abwandlung von den zuvor beschriebenen Ausführungen wäre es grundsätzlich auch möglich, die Pixelelemente 21 selbst als Referenzelemente 22 zu verwenden. Hierzu müßten dann die von den Pixelelementen 21 aufgenommenen Signale periodisch zur Mittelwertbildung integriert werden, der Mittelwert gespeichert werden und zur Steuerung des Verstärkungsfaktors in Abhängigkeit von dem gespeicherten Mittelwert verwendet werden. Die Mittelwertbildung müßte dann in vorgegebenen Zeitabständen wiederholt werden.

Durch eine derartige Anordnung ließe sich zwar die Auflösung noch weiter steigern bzw. der Aufbau des Chips 20 vereinfachen, da auf die Referenzelemente 22 verzichtet werden könnte, jedoch hätte dies zur Folge, daß zusätzliche Einrichtungen zur Mittelwertbildung und Speicherung und zur Zeittaktung vorgesehen werden müßten. Außerdem wäre das Implantat zu den Zeitpunkten, wenn die Mittelwertbildung und Auswertung aufgrund der von den Pixelelementen aufgenommenen Signale erfolgt, nicht aktiv, was zu einer gewissen Totzeit führen würde.

Dennoch wäre auch eine solche Ausführung natürlich grundsätzlich möglich.

## Patentansprüche

1. Retina-Implantat mit einer Oberfläche (27), an der eine Mehrzahl von Pixelelementen (21) zur Aufnahme und Umwandlung von einfallender Lichtenergie in elektrische Energie vorgesehen ist, mit mindestens einem Verstärker (24) und mit einer Mehrzahl von Stimulationselektroden (25), die über den mindestens einen Verstärker (24) in Abhängigkeit von den von den Pixelelementen (21) aufgenommenen Signalen angesteuert werden, **gekennzeichnet durch** mindestens ein lichtempfindliches Referenzelement (22, 22a, b, c), das eine größere Oberfläche aufweist als ein Pixelelement (21), oder **durch** eine Mehrzahl von Referenzelementen (22, 22a, b, c), wobei das Referenzelement (22, 22a, b, c) oder die Mehrzahl von Referenzelementen (22, 22a, b, c) mit dem mindestens einen Verstärker (24) gekoppelt ist, um dessen Verstärkung in Abhängigkeit von der auf das mindestens eine Referenzelement (22) einfallenden Lichtenergie zu steuern.

2. Retina-Implantat nach Anspruch 1, **dadurch gekennzeichnet, daß** ein gemeinsames, sich über einen ausgewählten Bereich der Oberfläche (27) erstreckendes Referenzelement (22) vorgesehen ist, auf dem Pixelelemente (21) als isolierte Bereiche, vorzugsweise in einem Gittermuster angeordnet sind.

3. Retina-Implantat nach Anspruch 1, **dadurch gekennzeichnet, daß** die Mehrzahl von Referenzelementen (22, 22a, b, c) über einen ausgewählten Bereich der Oberfläche (27) verteilt in einem vorgegebenen Muster zwischen den Pixelelementen (21) angeordnet ist.

4. Retina-Implantat nach Anspruch 3, **dadurch gekennzeichnet, daß** die Referenzelemente (22a, b, c) als Streifen und/oder als rechtwinkliges oder schiefwinkliges Gitternetz über einen ausgewählten Bereich der Oberfläche verteilt angeordnet sind.

5. Retina-Implantat nach Anspruch 4, **dadurch gekennzeichnet, daß** jeweils ein Referenzelement (22a) einer Mehrzahl von Pixelelementen (21) zugeordnet ist.

6. Retina-Implantat nach Anspruch 3, **dadurch gekennzeichnet, daß** jedem Pixelelement (21) ein Referenzelement zugeordnet ist.

7. Retina-Implantat nach. Anspruch 3, **dadurch gekennzeichnet, daß** einem Pixelelement (21) mehrere Referenzelemente (22c) zugeordnet sind.

8. Retina-Implantat nach einem der vorhergehenden Ansprüche, **dadurch gekennzeichnet, daß** jedem Pixelelement (21) ein Verstärker (24) und eine Stimulationselektrode (25) zugeordnet sind.

9. Retina-Implantat nach Anspruch 4 oder 5, **dadurch gekennzeichnet, daß** zwischen einzelnen Streifen (22a) und Pixelelementen (21) weitere Referenzelemente (22b) angeordnet sind.

10. Retina-Implantat nach Anspruch 8, **dadurch gekennzeichnet, daß** eine Mehrzahl von Referenzelementen (22c) schachbrettartig über einen ausgewählten Bereich der Oberfläche (27) verteilt angeordnet ist, wobei jedes Pixelelement (21) von mehreren Referenzelementen (22c) umgeben ist, die winklig zueinander angeordnet sind.

11. Retina-Implantat nach einem der vorhergehenden Ansprüche, **dadurch gekennzeichnet, daß** der mindestens eine Verstärker (24) die Differenz zwischen den Ausgangssignalen der Pixelelemente (21) und der zugeordneten Referenzelemente (22, 22a, b, c) verstärkt und die zugeordneten Stimulationselektroden (25) in Abhängigkeit von den verstärkten Signalen steuert.

12. Retina-Implantat nach einem der vorhergehenden Ansprüche, **dadurch gekennzeichnet, daß** die Pixelelemente (21) und das oder die Referenzelemente (22, 22a, b, c) eine gleiche Wellenlängenempfindlichkeit aufweisen.

13. Retina-Implantat nach einem der vorhergehenden Ansprüche, **dadurch gekennzeichnet, daß** die Pixelelemente (21) und das oder die Referenzelemente (22, 22a, b, c) als Photodioden ausgebildet sind.

14. Retina-Implantat nach einem der vorhergehenden Ansprüche, **dadurch gekennzeichnet, daß** die Pixelelemente (21) sowie das mindestens eine Referenzelement (22; 22a, 22b, 22c) jeweils eine vorbestimmte, dem einfallenden Licht zugewandte Oberfläche aufweisen, und daß die Oberfläche des Referenzelementes (22) bzw. die Summe der Oberflächen der Mehrzahl von Referenzelementen (22a, 22b, 22c) größer ist als die Oberfläche des diesen zugeordneten Pixelelementes (21).

15. Retina-Implantat nach einem der vorhergehenden Ansprüche, **dadurch gekennzeichnet, daß** die Pixelelemente (21) und das oder die Referenzelemente (22; 22a, 22b, 22c) als unterschiedliche Bauelemente ausgebildet sind.

16. Retina-Implantat nach einem der Ansprüche 1 bis 15, **dadurch gekennzeichnet, daß** die Pixelelemente und das oder die Referenzelemente jeweils dasselbe Bauelement sind.

## Claims

1. A retina implant, having a surface (27) with a plurality of pixel elements (21) disposed thereon for receiving and converting incoming light energy into electric energy, at least one amplifier (24) and a plurality of stimulation electrodes (25), said stimulation electrodes being controlled via the at least one amplifier (24) as a function of the signals received by the pixel elements (21), **characterized by** at least one light-sensitive reference element (22, 22a, b, c) having a surface that is larger than the surface of the pixel element (21) or by a plurality of reference elements (22, 22a, b, c), whereby the reference element (22, 22a, b, c) or the plurality of reference elements (22, 22a, b, c) are coupled with the at least one amplifier (24) for controlling the amplification thereof as a function of the light energy impinging on the at least one reference element (22).

2. The retina implant of claim 1, **characterized in that** a common reference element (22) is provided that extends over a selected portion of the surface (27), with pixel elements (21) being disposed on the reference element (22) as isolated areas, preferably along a grid pattern.

3. The retina implant of claim 1, **characterized in that** the plurality of reference elements (22, 22a, b, c) is distributed over a selected portion of the surface (27) along a predetermined pattern between the pixel elements (21).

4. The retina implant of claim 3, **characterized in that** the reference elements (22a, b, c) are distributed over a selected portion of the surface as stripes and/or as a rectangular or an oblique grid.

5. The retina implant of claim 4, **characterized in that** each one reference element (22a) each is associated to a plurality of pixel elements (21).

6. The retina implant of claim 3, **characterized in that** each pixel element (21) has one reference element associated thereto.

7. The retina implant of claim 3, **characterized in that** one pixel element (21) has a plurality of reference elements (22c) associated thereto.

8. The retina implant of any of the preceding claims, **characterized in that** each pixel element (21) has an amplifier (24) and a stimulation electrode (25) associated thereto.

9. The retina implant of claim 4 or 5, **characterized in that** further reference elements (22b) are disposed between individual stripes (22a) and pixel elements (21).

10. The retina implant of claim 8, **characterized in that** a plurality of reference elements (22c) is distributed over a selected portion of the surface (27) in a chessboard pattern, wherein each pixel element (21) is surrounded by a plurality of reference elements (22c) arranged at an angle with respect to each other.

11. The retina implant of any of the preceding claims, **characterized in that** the at least one amplifier (24) amplifies the difference between the output signals of the pixel elements (21) and of the associated reference elements (22, 22a, b, c), and controls the associated stimulation electrodes (25) as a function of the amplified signals.

12. The retina implant of any of the preceding claims, **characterized in that** the pixel elements (21) and the at least one reference element (22, 22a, b, c) have the same wavelength sensitivity.

13. The retina implant of any of the preceding claims, **characterized in that** the pixel elements (21) and the at least one reference element (22, 22a, b, c) are configured as photodiodes.

14. The retina implant of any of the preceding claims, **characterized in that** the pixel elements (21) as well as the at least one reference element (22; 22a, 22b, 22c) each have a predetermined surface facing the incoming light, and that the surface of the reference element (22) or the sum of the surfaces of the plurality of reference elements (22a, 22b, 22c), respectively, is larger than the surface of the pixel element (21) associated thereto.

15. The retina implant of any of the preceding claims, **characterized in that** the pixel element (21) and the at least one reference element (22; 22a, 22b, 22c) are configured as different components.

16. The retina implant of any of claims 1 to 15, **characterized in that** the pixel elements and the reference element or the reference elements are each the same component.

## Revendications

1. Implant rétinien présentant une surface (27) sur laquelle une pluralité de pixels (21) est prévue pour la réception et la conversion d'une énergie lumineuse incidente en énergie électrique, avec au moins un amplificateur (24) et avec une multiplicité d'électrodes de stimulation (25) qui sont commandées par au moins un amplificateur (24) en fonction des signaux enregistrés par les pixels (21), **caractérisé par** au moins un élément de référence (22, 22a, b, c) photosensible qui présente une surface supérieure à un pixel (21) ou par une pluralité d'éléments de référence (22, 22a, b, c), l'élément de référence (22, 22a, b, c) ou la pluralité d'éléments de référence (22, 22a, b, c) étant couplé(e) avec au moins un amplificateur (24), pour commander son amplification en fonction de l'énergie lumineuse incidente sur au moins un élément de référence (22).

2. Implant rétinien selon la revendication 1, **caractérisé en ce qu'**un élément de référence (22) commun, s'étendant sur une zone choisie de la surface (27) est prévu, sur lequel des pixels (21) sont disposés en tant que zones isolées, de préférence en un schéma en grille.

3. Implant rétinien selon la revendication 1, **caractérisé en ce que** la pluralité d'éléments de référence (22, 22a, b, c) est disposée sur une zone choisie de la surface (27), répartie en un schéma prédéterminé entre les pixels (21).

4. Implant rétinien selon la revendication 3, **caractérisé en ce que** les éléments de références (22a, b, c) sont disposés répartis en bandes et/ou en grillage à angle droit ou en biais, sur une zone choisie de la surface.

5. Implant rétinien selon la revendication 4, **caractérisé en ce que** respectivement, un élément de référence (22a) est affecté à une pluralité de pixels (21).

6. Implant rétinien selon la revendication 3, **caractérisé en ce que**, à chaque pixel (21) est affecté un élément de référence.

7. Implant rétinien selon la revendication 3, **caractérisé en ce que**, à un pixel (21) sont affectés plusieurs éléments de référence (22c).

8. Implant rétinien selon l'une des revendications précédentes, **caractérisé en ce que**, à chaque pixel (21) est affecté un amplificateur (24) et une électrode de stimulation (25).

9. Implant rétinien selon la revendication 4 ou 5, **caractérisé en ce que**, entre des bandes individuelles (22a) et des éléments à pixels (21), d'autres éléments de référence (22b) sont disposés.

10. Implant rétinien selon la revendication 8, **caractérisé en ce qu'**une pluralité d'éléments de référence (22c) est disposée répartie en damiers sur une zone choisie de la surface (27), chaque pixel (21) étant entouré de plusieurs éléments de référence (22c) qui sont disposés en angle l'un par rapport à l'autre.

11. Implant rétinien selon l'une des revendications précédentes, **caractérisé en ce qu'**au moins un amplificateur (24) amplifie la différence entre les signaux de sortie des pixels (21) et les éléments de référence affectés (22, 22a, b, c) et les électrodes de stimulation affectées (25) en fonction des signaux amplifiés.

12. Implant rétinien selon l'une des revendications précédentes, **caractérisé en ce que** les pixels (21) et le ou les élément(s) de référence (22, 22a, b, c) présentent une sensibilité de longueur d'onde égale.

13. Implant rétinien selon l'une des revendications précédentes, **caractérisé en ce que** les pixels (21) et le ou les élément(s) de référence (22, 22a, b, c) sont conçus comme des photodiodes.

14. Implant rétinien selon l'une des revendications précédentes, **caractérisé en ce que** les pixels (21) et au moins un élément de référence (22 ; 22a, 22b, 22c) présentent respectivement une surface prédéterminée tournée vers la lumière incidente et **en ce que** la surface de l'élément de référence (22) ou le total des surfaces de la pluralité des éléments de référence (22a, 22b, 22c) est supérieur(e) à la surface de ce pixel (21) tourné vers ceux-ci.

15. Implant rétinien selon l'une des revendications précédentes, **caractérisé en ce que** les pixels (21) et le ou les élément(s) de référence (22 ; 22a, 22b, 22c) sont conçus comme des éléments différents.

16. Implant rétinien selon l'une des revendications 1 à 15, **caractérisé en ce que** les pixels et le ou les éléments de référence sont respectivement le même élément.
